# EUROPEAN PATENT SPECIFICATION

(11) **EP 4 262 902 B1**
(45) Date of publication and mention of the grant of the patent: **29.01.2025**
(21) Application number: 21844198.8
(22) Date of filing: 14.12.2021
(51) Int. Cl.: A61F 13/00, A61F 13/0203, A61F 13/0206, A61F 13/02, A61K 31/198, A61K 31/785, A61L 15/28, A61L 15/46, C08L 1/28

(54) **METHOD FOR THE MANUFACTURE OF A WOUND CARE PRODUCT**
VERFAHREN ZUR HERSTELLUNG EINES WUNDPFLEGEPRODUKTS
PROCÉDÉ DE FABRICATION D'UN PRODUIT DE SOIN DES PLAIES

(30) Priority: 15.12.2020 DE 102020133583
(43) Date of publication of application: 25.10.2023
(60) Divisional application: 24222373.3
(73) Proprietor: Lohmann & Rauscher GmbH, 1140 Wien (AT)
(72) Inventor: LEEB, Tina, 2751 Matzendorf (AT); ROLJIC, Milijana, 2751 Steinabrückl (AT); PANHÖLZL, Christopher, 1170 Wien (AT); SCHIPALI, Stefanie, 200 Wien (AT); HARREITHER, Wolfgang, 2514 Traiskirchen (AT); BUCHEGGER, Patricia, 2801 Katzelsdorf (AT)
(74) Representative: Herrmann, Daniel
(86) International application number: PCT/EP2021/085616
(87) International publication number: WO 2022/128996

(56) References cited:
- EP-A1- 3 088 010
- EP-A1- 3 088 010
- WO-A1-03/022317
- WO-A1-03/022317
- US-A1- 2009 306 157
- US-A1- 2009 306 157
- US-A1- 2010 015 208
- US-A1- 2010 015 208
- US-A1- 2013 150 451
- US-A1- 2013 150 451
- US-A1- 2014 107 555
- US-A1- 2014 107 555
- KANTARAJA CHINDERA ET AL: "The antimicrobial polymer PHMB enters cells and selectively condenses bacterial chromosomes", SCIENTIFIC REPORTS, vol. 6, 21 March 2016 (2016-03-21), pages 23121, XP055326563, DOI: 10.1038/srep23121

## Description

The invention relates to a method for the manufacture of a wound care product whereby a fabric containing hydroactive constituents, in particular fibres, is processed.

Bandages in the form of nonwoven material, foams, sponges and cloths are used for the care of wounds. These are normally a fabric. In the context of this description and the claims, a fabric is understood to be a three-dimensional product which, when laid out flat, has an elongation in the thickness direction of 20% or less of the elongation in the longitudinal or transverse direction.

According to WO 2017/137419, the outcomes of wound care can be improved if a wound care product is saturated with an antimicrobial means such as an aqueous solution containing PHMB. Wound care products thus saturated allow germs to be killed off when caring for the wound. In the context of this description and the claims, any means by which germs can be killed off is described as antimicrobial, and a germ is understood to be any microorganism that causes illness (pathogen).

According to another treatment concept, wound care products with hydroactive constituents are used. In the context of this description and the claims, constituents are described as hydroactive if they form a gel on contact with water. The water in the gel may form bonds by means of hydrogen bonding. If a wound care product has hydroactive constituents, a desired wound climate which prevents both excessive drying of the wound and maceration of the wound can be created, particularlyin the treatment of acute and chronic wounds. In the manufacture of wound care products containing hydroactive constituents, fabrics in the form of woven or nonwoven material or even sponges with hydroactive constituents can be manufactured and subsequently cut to the specified size and then packed in a sterile pack.

When wound care products containing hydroactive constituents and manufactured in the conventional method are used, however, it has been found in the treatment of acute wounds that in many cases the wound is only observed to heal slowly, while in the treatment of chronic wounds the outcomes in some cases are not very satisfactory.

Methods for the manufacture of wound care products, in which antimicrobial means are applied onto hydroactive constituents of a fabric, are disclosed in US 2010/015208 A1, US 2009/306157, US 2014/107555 A1, EP 3 088 010 A1 and WO 03/022317. Further, US 2013/150451 A1 discloses an antimicrobial composition with synergistic biocidal activity.

In view of these problems in the state of the art, the object of the invention is to provide wound care products in the form of fabrics containing hydroactive constituents with which satisfactory outcomes in the treatment of acute and chronic wounds can be obtained.

According to the invention, this object is achieved by a further development of the known methods as specified in the characterizing portion of claim 1.

The invention results from the surprising discovery that hydroactive constituents of a wound care product which are provided with an antimicrobial means as specified in claim 1 release the antimicrobial constituents on contact with the wound fluid, before a gel is formed in which the fluid is bound. This result is unexpected because a stabilisation and hence deactivation of the antimicrobial means on contact with wound fluid or water as part of the gel formation of the hydroactive constituents of the wound care product seemed more likely. The release that is actually observed of the antimicrobial means applied to the hydroactive constituents of the fabric when wound care products manufactured according to the invention are used enables germs in the wound to be killed off and hence improves healing of the wound.

Surprisingly, it has been found that the desired release of the antimicrobial means applied to the hydroactive constituents is encouraged if this antimicrobial means is applied to the fabric in a preferably aqueous solution. A solution containing the antimicrobial means can be sprayed preferably on respective sides, that is both sides of the fabric, whereby in the context of the invention it is particularly preferred if the solution is sprayed with at least one ultrasonic nozzle. In the context of this description and the claims, ultrasonic nozzles in which high-frequency oscillations are used to act on the nozzle tip and generate capillary waves in a fluid film are described as such spray nozzles. As soon as the amplitude of the capillary waves reaches a critical height, these waves become too great to support themselves. This causes tiny droplets to fall from the tip of the individual waves, which leads to atomisation. The main factors influencing the initially generated droplet size are the oscillation frequency, the surface tension and the viscosity of the fluid. In comparison to a pneumatic spray system, the ultrasonic spray system allows the droplet size, the distribution and the speed to be better adjusted.

If an aqueous solution of the antimicrobial means is sprayed onto the hydroactive constituents, droplet sizes can be set so that sufficiently low quantities of the water used as the solvent evaporate, meaning that an early swelling or early gel formation of the hydroactive constituent of the fabric can be suppressed. This simultaneously has the effect that although the antimicrobial means are bonded to the hydroactive constituents, the bonding forces remain so low that, contrary to expectation, on contact with wound fluid or water a release of the antimicrobial means occurs before a gel is formed under the influence of the wound exudate.

Especially in the case of those fabrics which consist of hydroactive constituents to more than 50% by weight, in particular about 80% by weight, it has proved to be particularly expedient in the context of the invention if the antimicrobial means is applied in a quantity of 10⁻⁴ mg or more, in particular 4*10⁻³ mg or more, per cm² of a respective side of the fabric. If the antimicrobial means is applied in a lower quantity, the desired antimicrobial effect is hardly observed.

Having regard to the stability of the aqueous solution used to apply the antimicrobial means, it has proved to be expedient if the antimicrobial means is applied in a quantity of 1.0 mg or less, in particular 0.7 mg or less, per cm² of a respective side of the fabric. A greater quantity of the antimicrobially effective means can hardly be applied because the solubility of conventional antimicrobial means such as PHMB (polyhexanide) is limited in water and application of the antimicrobial means in a larger quantity would therefore be associated with a greater ingress of fluid into the hydroactive constituents of the fabric, which would in turn lead to premature gel formation.

The antimicrobial means in the implementation of the method according to the invention comprises PHMB. The PHMB in an aqueous solution is present in a concentration in the range of 0.1 to 9% by weight, in particular in the range of 4 to 9% by weight, whereby preferably 0.1 to 8 mg, in particular 2 to 8 mg, of the solution is applied per cm² to a respective side of the fabric. If PHMB is present in a lower concentration, the application of sufficient quantities onto the hydroactive constituents can require too much fluid, which can lead to premature gel formation. If the concentration of PHMB in the solution exceeds 9% by weight, the solution begins to become unstable overall.

In many cases, the germs that impair the healing of chronically infected wounds exist as what are known as biofilms. The extracellular polymeric substances (EPS) that are excreted combine with water to form hydrogels, so that a mucous matrix in which nutrients and other substances are dissolved is created. Often inorganic particles or gas bubbles are also enclosed by the matrix. In this form the germs are protected from external influences such as attacks by the immune system or antibiotics or antimicrobial means. Having regard to the protection of the germs by the extracellular matrix, a wound care product that is to act against the biofilms must meet the following requirements:
1. Break up the extracellular matrix
2. Kill off the germs

Since conventional antimicrobial means manifest only deficient properties for breaking up the extracellular matrix, it has proved to be particularly expedient for the invention if, in addition to the antimicrobial means, a chelator is also applied to the hydroactive constituents when wound care products containing hydroactive constituents are manufactured.

The chelator enables the extracellular matrix to be weakened, in particular through the bonding of cations, so that the germs living in the matrix can be killed off with the help of the antimicrobial means. In the context of the invention, the chelator is preferably applied in the preferably aqueous solution also containing the antimicrobial means, in particular sprayed onto the hydroactive constituents of the fabric, more preferably sprayed on with at least one ultrasonic nozzle.

The chelator is expediently applied in a quantity of 5*10⁻⁴ mg or more, in particular 10⁻² mg or more, per cm² of a respective side of the fabric. If the quantity of chelator is less, the desired matrix-weakening effect will hardly be achieved. It was also observed that a further improvement of the weakening effect on the matrix of the biofilm will hardly be achieved if the chelator is applied in a quantity of more than 1.2 mg per cm² of a respective side of the fabric. For this reason it has also proved to be expedient in the context of the invention if the chelator is applied in a quantity of 1.2 mg or less, in particular 0.8 mg or less, per cm² of a respective side of the fabric. In this regard also the stated limit values are in particular preferred in such fabrics in which more than 50% by weight of the fabric is formed by the hydroactive constituents, in particular if the hydroactive constituents form about 80% by weight of the fabric.

According to the invention the chelator comprises EDTA (ethylenediaminetetraacetic acid), in particular a disodium salt of EDTA (ethylenediaminetetraacetic acid disodium salt dihydrate (C₁₀H₁₄N₂Na₂O₈ 2H₂O)).

The disodium salt has proved to be particularly preferable in the context of the invention. With this salt of ethylenediaminetetraacetic acid, a pH value of less than 7 can be set in the aqueous solution with the desired solubility (high concentration). With other EDTA variants, higher pH values are normally observed alongside lower solubility. A colour change is also achieved if the final products are stored at temperatures above 40° C. The setting of a low pH value strengthens the desired antimicrobial effect. In addition, if EDTA is used, in particular the disodium salt thereof, an independent antimicrobial effectiveness of this chelator is also observed. EDTA can have the effect of killing off those germs against which PHMB has only a somewhat lesser effect. The main purpose of the addition of EDTA in the solution used for the manufacture of wound care products according to the invention is, however, the effect it has of weakening the matrix and stabilising the formulation and process. According to this invention, it has been proved beneficial in relation both to the application of antimicrobial means and chelators on hydroactive constituents of the fabric and to the stabilisation of the aqueous solution manufactured using the method according to the invention for application of the stated substances, that in addition to the antimicrobial means, and the chelator EHG (ethylhexyl monoglycerine ether) and a polysorbate i. e. Tween 20 is also applied onto the hydroactive constituents. The tenside in the preferably aqueous solution can be applied, in particular sprayed on, more preferably sprayed on with the aid of at least one ultrasonic nozzle.

In the context of the invention, particularly if fabrics of which more than 50% by weight is comprised of hydroactive constituents are used, the tenside is applied preferably in a quantity of 10⁻⁴ mg or more, in particular 5*10⁻⁴ mg or more, more preferably 10⁻³ mg or more, per cm² of a respective side of the fabric. If at least one tenside is applied in a lower quantity, the desired effect is no longer achieved. To avoid the inactivation of other substances of the solution, and having regard to biocompatibility, particularly having regard to avoiding the formation of formaldehyde, it is more preferred in the context of the invention if the tenside is applied in a quantity of 0.6 mg or less, in particular 0.1 mg or less, per cm² of a respective side of the fabric.

In the implementation of the method according to the invention, the tenside in the aqueous solution can be present in a concentration in the range of 0.1 to 7.5% by weight, in particular 0.1 to 5% by weight, whereby preferably 0.1 to 8 mg, in particular 2 to 8 mg of the aqueous solution, is applied per cm² to a respective side of the fabric. If EHG is used as a tenside, the concentration of this tenside in the aqueous solution is preferably between 0.1 and 5% by weight. Tween 20 is added to the aqueous solution as an additional tenside, wherein the concentration of this tenside is 0.5 to 2.5% by weight. The addition of a higher concentration of EHG can lead to the formation of formaldehyde. The addition of a higher concentration of Tween 20 can lead to an inactivation of the antimicrobial means, such as PHMB.

If the tensides are applied in a smaller quantity than indicated, this leads to a loss of regularity in the spraying process. A loss of the stabilisation effect of the solution is also observed. Both EHG and Tween 20 manifest an antimicrobial effect in addition to stabilising the solution. As soon as the concentration falls below the indicated lower limits, this leads to a loss of the antimicrobial effect. Furthermore, in the case of Tween 20 a destabilising effect on the extracellular matrix of the biofilm is also observed. This effect is no longer observed if the quantity of Tween 20 in the aqueous solution falls below 0.5% by weight.

An aqueous solution for the implementation of the method according to the invention preferably comprises the following constituents:
1. PHMB: 0.1 - 9% by weight, preferably 4 - 9% by weight
2. EDTA: 0.5 - 15% by weight, preferably 5 - 10.4% by weight
3. EHG: 0.1 - 5% by weight, preferably 2 - 5% by weight
4. Tween 20: 0.5 - 2.5% by weight
5. Water: rest and unavoidable contamination

In the implementation of the method according to the invention, it has surprisingly been found that the desired success in the treatment of chronic wounds with the formation of a biofilm is achieved particularly well if the proportion of the concentration of the antimicrobial means such as PHMB in the aqueous solution relative to the concentration of the chelator such as EDTA disodium salt in the aqueous solution is 0.6 or more. The relatively high proportion of the antimicrobial means such as PHMB leads, on contact of the hydroactive constituent of the fabric with water, to the release of a sufficient quantity of the antimicrobial means which is available after the matrix of the biofilm has been weakened by the chelator for killing off the germs.

In view of the known compositions of aqueous solutions used for saturating conventional bandages, this too is surprising. With these known solutions a much greater proportion of chelator is required. The advantages arising in the method according to the invention from setting the proportions of the concentration of the antimicrobial means relative to the concentration of the chelator in the aqueous solution can be explained in that the chelator is surprisingly better released from the hydroactive constituent of the fabric on contact with water or wound exudate than the antimicrobial means.

In all embodiments of the invention, it is particularly preferred if the aqueous solution used to apply the antimicrobial means during the spraying process is held in a container which is kept at a constant temperature of between 35°and 70° C in order to increase solubility and in which the aqueous solution is continuously agitated.

A wound care product obtained by a method according to the invention having a fabric comprising hydroactive constituents is essentially characterised in that the hydroactive constituents are provided with an antimicrobial means. In preferred embodiments of wound care products according to the invention, the quantity of the antimicrobially effective means is between 10⁻⁴ mg and 1.0 mg, preferably between 4*10⁻³ mg and 0.7 mg, per cm² of a respective side of the fabric. The antimicrobial means can comprise PHMB. In addition, a wound care product according to the invention can also be provided with a chelator in the area of its hydroactive constituents. The quantity of chelator is preferably 5*10⁻⁴ mg to 1.2 mg, in particular 10⁻² to 0.8 mg, per cm² of a respective side of the fabric. In the context of the invention, the chelator of a wound care product according to the invention is an EDTA disodium salt.

In addition or as an alternative to the chelator, the hydroactive constituent of the wound care product according to the invention can also be provided with a tensidei. e. EHG or a polysorbate, which can be applied in a quantity of 10⁻⁴ to 0.6 mg, in particular 5*10⁻⁴ to 0.1 mg, per cm² of a respective side of the fabric. The fabric of a wound care product according to the invention is preferably comprised of the hydroactive constituent to 50% by weight or more, in particular about 80% by weight of the hydroactive constituent.

The hydroactive constituents of the wound care product according to the invention are cellulose ethyl sulphonate fibres and/or carboxymethyl cellulose fibres. The fabric can additionally also comprise cellulose fibres.

## Claims

1. Method for the manufacture of a wound care product in which a fabric containing hydroactive constituents is processed, wherein said hydroactive constituents are carboxymethyl cellulose fibres and/or cellulose ethyl sulphonate fibres, wherein the processing of the fabric comprises the application of an antimicrobial means onto the hydroactive constituents, **characterised in that** the antimicrobial means is applied in an aqueous solution comprising 0.1 to 9% by weight of polyhexanide (PHMB), 0.5 to 15% by weight of EDTA disodium salt, 0.1 to 5% by weight of ethylhexyl monoglycerine ether (EHG), and 0.5 to 2.5% by weight of Tween 20.

2. Method according to claim 1, **characterised in that** the solution containing the antimicrobial means is sprayed preferably onto both sides of the fabric, in particular with at least one ultrasonic nozzle.

3. Method according to claim 1 or 2, **characterised in that** 10⁻⁴ mg or more, in particular 4*10⁻³ mg or more, of the antimicrobial means is applied per cm² of a respective side of the fabric.

4. Method according to one of claims 1 to 3, **characterised in that** 1.0 mg or less, in particular 0.7 mg or less, of the antimicrobially effective means is applied per cm² of a respective side of the fabric.

5. Method according to any of the previous claims, **characterised in that** PHMB is present in the aqueous solution in a concentration in the range of 4 to 9% by weight, whereby preferably 0.1 to 8 mg, in particular 2 to 8 mg, of the solution is applied per cm² to a respective side of the fabric.

6. Method according to any of the previous claims, **characterised in that** EDTA is present in the aqueous solution in a concentration in the range of 5 to 10.4% by weight, whereby preferably 0.1 to 8 mg, in particular 2 to 8 mg, of the solution is applied per cm² to a respective side of the fabric.

7. Method according to any of the previous claims, **characterised in that** 10⁻⁴ mg or more, in particular 5*10⁻⁴ mg or more, more preferably 10⁻³ mg or more, of EHG is applied per cm² of a respective side of the fabric.

8. Method according to any of the previous claims, **characterised in that** 0.6 mg or less, in particular 0.1 mg or less, of EHG is applied per cm² of a respective side of the fabric.

9. Method according to one of the previous claims, **characterised in that** the proportion of hydroactive constituents in the fabric is 50% or more by weight, in particular 75% or more by weight, more preferably about 80% by weight.

## Patentansprüche

1. Verfahren zur Herstellung eines Wundpflegeprodukts, bei dem ein Gewebe, das hydroaktive Bestandteile enthält, verarbeitet wird, wobei die hydroaktiven Bestandteile Carboxymethylcellulosefasern und/oder Celluloseethylsulfonatfasern sind, wobei die Verarbeitung des Gewebes das Aufbringen eines antimikrobiellen Mittels auf die hydroaktiven Bestandteile umfasst, **dadurch gekennzeichnet, dass** das antimikrobielle Mittel in einer wässrigen Lösung aufgebracht wird, die 0,1 bis 9 Gew.-% Polyhexanid (PHMB), 0,5 bis 15 Gew.-% EDTA-Dinatriumsalz, 0,1 bis 5 Gew.-% Ethylhexylmonoglycerinether (EHG) und 0,5 bis 2,5 Gew.-% Tween 20 umfasst.

2. Verfahren nach Anspruch 1, **dadurch gekennzeichnet, dass** die Lösung, die das antimikrobielle Mittel enthält, vorzugsweise auf beide Seiten des Gewebes gesprüht wird, insbesondere mit mindestens einer Ultraschalldüse.

3. Verfahren nach Anspruch 1 oder 2, **dadurch gekennzeichnet, dass** 10⁻⁴ mg oder mehr, insbesondere 4*10⁻³ mg oder mehr, des antimikrobiellen Mittels pro cm2 einer jeweiligen Seite des Gewebes aufgebracht werden.

4. Verfahren nach einem der Ansprüche 1 bis 3, **dadurch gekennzeichnet, dass** 1,0 mg oder weniger, insbesondere 0,7 mg oder weniger, des antimikrobiell wirksamen Mittels pro cm2 einer jeweiligen Seite des Gewebes aufgebracht werden.

5. Verfahren nach einem der vorhergehenden Ansprüche, **dadurch gekennzeichnet, dass** PHMB in der wässrigen Lösung in einer Konzentration im Bereich von 4 bis 9 Gew.-% vorhanden ist, wobei vorzugsweise 0,1 bis 8 mg, insbesondere 2 bis 8 mg, der Lösung pro cm2 auf eine jeweilige Seite des Gewebes aufgebracht werden.

6. Verfahren nach einem der vorhergehenden Ansprüche, **dadurch gekennzeichnet, dass** EDTA in der wässrigen Lösung in einer Konzentration im Bereich von 5 bis 10,4 Gew.-% vorhanden ist, wobei vorzugsweise 0,1 bis 8 mg, insbesondere 2 bis 8 mg, der Lösung pro cm2 auf eine jeweilige Seite des Gewebes aufgebracht werden.

7. Verfahren nach einem der vorhergehenden Ansprüche, **dadurch gekennzeichnet, dass** 10⁻⁴ mg oder mehr, insbesondere 5*10⁻⁴ mg oder mehr, bevorzugter 10⁻³ mg oder mehr, EHG pro cm2 einer jeweiligen Seite des Gewebes aufgebracht werden.

8. Verfahren nach einem der vorhergehenden Ansprüche, **dadurch gekennzeichnet, dass** 0,6 mg oder weniger, insbesondere 0,1 mg oder weniger, EHG pro cm2 einer jeweiligen Seite des Gewebes aufgebracht werden.

9. Verfahren nach einem der vorhergehenden Ansprüche, **dadurch gekennzeichnet, dass** der Anteil an hydroaktiven Bestandteilen im Gewebe 50 Gew.-% oder mehr, insbesondere 75 Gew.-% oder mehr, bevorzugter etwa 80 Gew.-% beträgt.

## Revendications

1. Procédé de fabrication d'un produit de soin des plaies, dans lequel un tissu contenant des constituants hydroactifs est traité, dans lequel lesdits constituants hydroactifs sont des fibres de carboxyméthylcellulose et/ou des fibres d'éthylsulfonate de cellulose, dans lequel le traitement du tissu comprend l'application d'un agent antimicrobien sur les constituants hydroactifs, **caractérisé en ce que** l'agent antimicrobien est appliqué dans une solution aqueuse comprenant 0,1 à 9 % en poids de polyhexanide (PHMB), 0,5 à 15 % en poids de sel disodique d'EDTA, 0,1 à 5 % en poids d'éther monoglycérinique d'éthylhexyle (EHG) et 0,5 à 2,5 % en poids de Tween 20.

2. Procédé selon la revendication 1, **caractérisé en ce que** la solution contenant l'agent antimicrobien est pulvérisée de préférence sur les deux côtés du tissu, en particulier avec au moins une buse à ultrasons.

3. Procédé selon la revendication 1 ou 2, **caractérisé en ce que** 10⁻⁴ mg ou plus, en particulier 4*10⁻³ mg ou plus, de l'agent antimicrobien sont appliqués par cm² d'un côté respectif du tissu.

4. Procédé selon l'une des revendications 1 à 3, **caractérisé en ce que** 1,0 mg ou moins, en particulier 0,7 mg ou moins, de l'agent antimicrobien efficace sont appliqués par cm² d'un côté respectif du tissu.

5. Procédé selon l'une quelconque des revendications précédentes, **caractérisé en ce que** le PHMB est présent dans la solution aqueuse à une concentration dans la plage de 4 à 9 % en poids, où de préférence 0,1 à 8 mg, en particulier 2 à 8 mg, de la solution sont appliqués par cm² d'un côté respectif du tissu.

6. Procédé selon l'une quelconque des revendications précédentes, **caractérisé en ce que** l'EDTA est présent dans la solution aqueuse à une concentration dans la plage de 5 à 10,4 % en poids, où de préférence 0,1 à 8 mg, en particulier 2 à 8 mg, de la solution sont appliqués par cm² d'un côté respectif du tissu.

7. Procédé selon l'une quelconque des revendications précédentes, **caractérisé en ce que** 10⁻⁴ mg ou plus, en particulier 5*10⁻⁴ mg ou plus, plus préférablement 10⁻³ mg ou plus, d'EHG sont appliqués par cm² d'un côté respectif du tissu.

8. Procédé selon l'une quelconque des revendications précédentes, **caractérisé en ce que** 0,6 mg ou moins, en particulier 0,1 mg ou moins, d'EHG sont appliqués par cm² d'un côté respectif du tissu.

9. Procédé selon l'une quelconque des revendications précédentes, **caractérisé en ce que** la proportion de constituants hydroactifs dans le tissu est de 50 % ou plus en poids, en particulier de 75 % ou plus en poids, plus préférablement d'environ 80 % en poids.
